# EUROPEAN PATENT APPLICATION

(11) **EP 2 692 722 A1**
(43) Date of publication of application: **05.02.2014**
(21) Application number: 11862518.5
(22) Date of filing: 02.11.2011
(51) Int. Cl.: C07C 321/22, C07C 323/22, C07C 319/12, C07C 323/52

(54) **SULFUR-CONTAINING FULLERENE DERIVATIVES AND METHOD FOR PRODUCING SAID DERIVATIVES**

(30) Priority: 30.03.2011 RU 2011111783
(71) Applicant: Uchrezhdenie Rossiiskoi Akademii Nauk Institut Problem Khimicheskoi Fiziki Ran (IPKHF RAN), Chernogolovka, Moscovskaya obl. 142432 (RU)
(72) Inventor: TROSHIN, Pavel Anatolievich, Chernogolovka Moskovskaya obl. 142432 (RU); RAZUMOV, Vladimir Fedorovich, Chernogolovka Moskovskaya obl. 142432 (RU); KHAKINA, Ekaterina Aleksandrovna, Moscow 125480 (RU)
(74) Representative: Jansen, Cornelis Marinus
(86) International application number: PCT/RU2011/000850
(87) International publication number: WO 2012/134337

(57) **Abstract**

The invention relates to the chemical and pharmaceutical branches of industry and concerns the chemical functionalization of fullerene C₆₀, and specifically the development of a method for producing a new class of sulfur-containing fullerene C₆₀ derivatives.

The problem addressed by the invention is that of expanding the range of fullerene derivatives.

The stated problem is solved by a new class of sulfur-containing fullerene derivatives of general formula 1, and specifically: 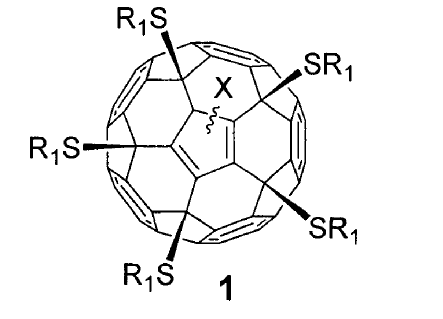 and by a method for producing said derivatives.

The invention can be used in biomedical investigations and in pharmacology, and also in organic photovoltaics.

## Description

The invention relates to the chemical and pharmaceutical branches of industry and concerns the chemical functionalization of fullerene C₆₀, and specifically the development of a method for producing a new class of sulfur-containing fullerene C₆₀ derivatives. The invention may find application in the biomedical and pharmaceutical research and in the development of organic photovoltaic promising light-converting material for organic solar batteries.

Fullerenes were discovered in 1985 in the mass spectrometric analysis of the products of laser vaporization of graphite [W. Kroto, J. R. Heath, S. C. O'Brien, R. F. Curl, R. E. Smalley. C60: Buckminsterfullerene, Nature, 318, 162-163 (1985)]. Following the development of methods for the preparation of fullerenes in macroscopic quantities by arc evaporation of graphite [W. Krätchmer, L. D. Lamb, K. Fostiropoulos, D. R. Huffman. C60: a new form of carbon. Nature, 347, 354-358 (1990)], or in the incomplete combustion of hydrocarbons [H. Murayama, S. Tomonoh, J. M. Alford, M. E. Karpuk. Fullerene production in tons and more: From science to industry. Full. Nanotub. Carbon Nanostruct., 12, 1-9 (2004)] and the effective separation methods of fullerenes [K. Nagata, E. Dejima, Y. Kikuchi, M. Hashiguchi. Kilogram-scale [60] fullerene separation from a fullerene mixture: Selective complexation of fullerenes with 1,8-diazabicyclo[5.4.0]undec-7-ene (DBU). Chem. Lett., 34, 178-179 (2005)] fullerene C₆₀ lower high purity became available in kilogram quantities for less than 15 €/g [http://www.neotechproduct.ru/str6.php?lang=rus].

After many years of research on fullerenes and their derivatives there were found among them compounds having valuable properties, which may find practical application. So, the derivatives of fullerene C₆₀ and C₇₀ are promising materials for organic solar cell which efficiency reaches 7-8% [Y. Liang, Z. Xu, J. Xia, S.-T. Tsai, Y. Wu, G. Li, C. Ray, L. Yu, For the Bright Future-Bulk Heterojunction Polymer Solar Cells with Power Conversion Efficiency of 7.4%. Adv. Mater. 22, 1-4 (2010)], as well as essential components of field-effect transistors [T. D. Anthopoulos, S. Tanase et al. Ambipolar Organic Field-Effect Transistors Based on a Solution-Processed Methanofullerene. Adv. Mater., 16, 2174 (2004)]. Moreover, some derivatives of fullerenes may be used as high temperature superconductors [M. J. Rosseinsky. Recent developments in the chemistry and physics of metal fullerides. Chem. Mater., 10, 2665-2685 (1998)], proton conductors [R. Maruyama. Electrochemical mass-flow control of hydrogen using a fullerene-based proton conductor. Electrochemica Acta, 48, 85-89 (2002)], nonlinear optical materials [L. W. Tutt, A. Kost. Optical limiting performance of C60 and C70 solutions. Nature, 356, 225-226 (1992)], etc.

One of the most important prospects of practical application of fullerene compounds is their use in medical research. Up-to-date, known are water-soluble fullerene derivatives, mainly containing ionic groups - amine [A. O. Troshina, P. Troshin, A. Peregudov, V. Kozlovski, R. Lyubovskaya. Photoaddition of N-Substituted Piperazines to C60: An Efficient Approach to the Synthesis of Water-Soluble Fullerene Derivatives. Chem. Eur. Journal, 12, 5569-5577 (2006)] and carboxyl [M. Brettereich, A. Hirsch. A highly water-soluble dendro[60]fullerene. Tetrahedron Letters, 39, 2731-2734 (1998) 13 - O. Troshina, P. Troshin, A. Peregudov, V. Kozlovskiy, J. Balzarini, R. Lyubovskaya. Chlorofullerene C60Cl6: a precursor for straightforward preparation of highly water-soluble polycarboxylic fullerene derivatives active against HIV. Org. Biomol. Chem., 5, 2783-2791 (2007)*]*, *which are potential antiviral (including anti-HIV)* [S. Wilson in ("Perspectives of Fullerene Nanotechnology", E. Osawa Ed. 2001, Kluver Academ; 15 - RF Patent Nº RU 2236852 C1 "Means for inhibiting reproduction of enveloped viruses, the method of its preparation, pharmaceutical composition and method of inhibiting viral infections", JSC "Desko", director L. D. Rasnetsov] and neuroprotective drugs [A. Lin, S. Fang, S. Lin, S. Chou, T. Luh, L. Ho. Local carboxyfullerene protects cortical infarction in rat brain. NeuroScience Research, 43, 317-321 (2002)]. Several fullerene derivatives showed high antitumor activity in photodynamic [Y. Tabata, T. Ishii, T. Aoyama, R. Oki, Y. Hirano, A. Ogawa, Y. Ikada in ("Perspectives of Fullerene Nanotechnology", E. Osawa (Ed.) 2001, Kluver Academ. Publ., Dordrecht-Boston-London.; Andreev S. M., Laptev V. P., Panferova N. G., Romanov V. S., Petrov, V. V., Ovchinnikov A. E., RF Patent RU 2005140680 A "Pharmaceutical composition for photodynamic therapy and method for the treatment of cancer with its use"] and chemical [K. YASUHIKO, O. KENK, IDEAL STAR INC, patent JP2005053904 (A) "Fullerene and anticancer therapeutic agent"; Patent NºPCT/RU2007/000337. 2007 "Multifunctional amino acid derivatives of fullerene C₆₀, containing biologically active groups, peptides or proteins, methods of making (options), the use of as a donor of nitric oxide and as a vasodilator, as well as a method of inhibiting the process of metastasis". Kotelnikov A. I., Romanova V. S, Bogdanov, G. N., Konovalova N. P., Kotelnikova R. A., Finegold I. L., Frog E. S., Pisarenko O. I., Bubnov Yu. N., Davydov M. I., Aldoshin S. M.] therapy of cancer. Thus, from all the above it can be concluded about the great practical value of the fullerene derivatives.

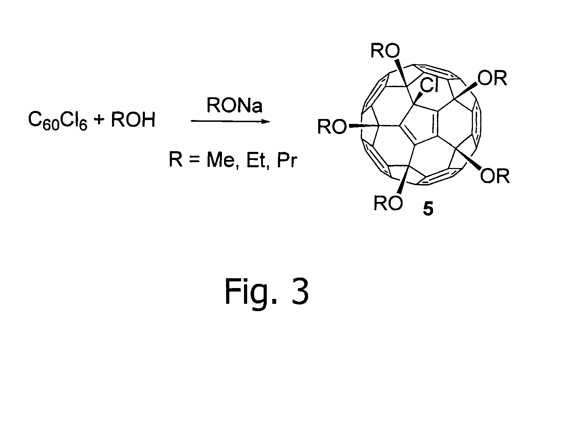

The prototype of the claimed method for producing sulfur-containing fullerene derivatives is a method according to which chlorfullerene C₆₀Cl₆ reacts with alcohols, the reaction proceeds in the presence of an appropriate sodium alcoholate as a base (Fig. 3) [A. G. Avent, P. R. Birkett, A. D. Darwish, S. Houlton, R. Taylor, K. S. T. Thomson, X. Wei, "Formation and characterization of alkoxy derivatives of [60]fullerene", J. Chem. Soc, Perkin Trans. 2, 2001, 782-786]. This reaction leads to the fullerene derivative with five attached alkoxy groups, which makes it similar to the compounds of general formula 1. The yield of the product of the reaction is low (less than 60 percent). It is also possible to note the low reaction rate (over one hour).

The problem of the invention is to expand the arsenal of fullerene derivatives.

The problem stated is solved by a new class of sulfur-containing fullerene derivatives of general formula 1, namely: 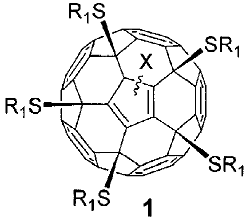 wherein in general formula, X 1 is, but not limited to:
- negative charge («-»), which is localized on the fullerene 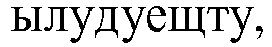 or
- chlorine atom (-C1) connected to the carbon skeleton, or
- nitro group (-NO₂) and nitrate group (-ONO₂), attached to the carbon skeleton, or
- the hydrogen atom (-H), linear or branched alkyl (CₙH₂ₙ₊₁; n = 1-20), alkenyl (CₙH₂ₙ₋₁; n=1-20) or alkynyl radical (CₙH₂ₙ₋₃, n=1 -20) attached to the fullerene skeletone, or
- alkoxyl O-R or thiolate moiety S-R, connected to the fullerene skeleton, wherein R is a hydrogen atom or a linear or branched alkyl (CₙH_{2n +1} and n= 1-20), alkenyl (CₙH₂ₙ₋₁; n=1 -20) or alkynyl radical (CₙH₂ₙ₋₃, n=1-20), or
- the residue of thioacid -S(CH₂)ₙCOOH or an ester thereof -S(CH₂)ₙCOOR, or an amide - S(CH₂)ₙCONR¹R² attached to the fullerene skeleton, where n=1-20, and R, R¹ and R² are hydrogen atoms or linear or branched alkyl (CₘH₂ₘ₊₁; n=1-20), alkenyl (CₘH₂ₘ₋₁; n=1-20) or alkynyl (CₘH₂ₘ₋₃, and n=1-20) radicals;
wherein in the general formula 1 the SR¹ fragment is described, but not limited to the following definitions:
- the residue of thioacid or ester thereof, wherein R¹ is given as XCOOR; X is a linear or branched alkylene (CₙH₂ₙ, n=1-20), alkenylene (CₙH₂ₙ₋₂, n=1-20) or alkynylene (CₙH₂ₙ₋₄; n=1-20) radical, as well as saturated (CₙH₂ₙ₋₂, n=3-8), unsaturated (CₙH₂ₙ₋₄; n=5-8, CₙH₂ₙ₋₆; n=5-8, CₙH₂ₙ₋₈, n=8) or an aromatic ring; R is a hydrogen atom (-H), a linear or branched alkyl (CₙH₂ₙ₊₁; n=1-20), alkenyl (CₙH₂ₙ₋₁; n=1-20) or alkynyl radical (CₙH₂ₙ₋₃, and n=1-20);
- the residue of the thioacid amide, where R¹ is given as XCONRR'; X is a linear or branched alkylene (CₙH₂ₙ; n=1-20), alkenylene (CₙH₂ₙ₋₂, n=1-20) or alkynylene (CₙH₂ₙ₋₄; n=1-20) radical, as well as saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or an aromatic ring; R and R' are hydrogen atoms or linear or branched alkyl (CₙH₂ₙ₊₁; n=1-20), alkenyl (CₙH₂ₙ₋₁; n=1-20) or alkynyl (CₙH₂ₙ₋₃; and n=1-20) radicals;
- the residue of the thiol, where R¹ is a hydrogen atom or a linear or branched alkyl (CₙH₂ₙ₊₁; n=1-20), alkenyl (CₙH₂ₙ₋₁; n=1-20) or alkynyl radical (CₙH₂ₙ₋₃; n=1-20) as well as saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or an aromatic ring;
- moiety of the general formula-S(CH₂₎ₙSR, wherein R is a hydrogen atom or a linear or branched alkyl (CₙH₂ₙ₊₁; n=1-20), alkenyl (CₙH₂ₙ₋₁; n=1-20) or alkynyl radical (CₙH₂ₙ₋₃; n=1-20) and saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or an aromatic ring;
- moiety of the general formula-S(CH₂)ₙHal, where Hal is F, CI, Br or I;
- moiety of the general formula-S(CH₂₎NRR' or -S(CH₂₎ₙN⁺RR'R" X⁻, where R, R' and R" are hydrogen atoms or linear or branched alkyl (CₙH₂ₙ₊₁; n=1-20), alkenyl (CₙH₂ₙ₋₁; n=1-20) or alkynyl (CₙH₂ₙ₋₃, n=1-20) radicals, as well as saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or aromatic rings; X⁻ is any anion.

Moreover, the stated problem is achieved by method for producing sulfur-containing fullerene derivatives of general formula 1 which comprises reacting chlorfullerene C₆₀Cl₆ with a nucleophile agent in the presence of a base, wherein as the nucleophile agent derivatives of thioacids, thioacids or thiols are taken. The process is conducted at a temperature of 0-150°C, preferably at 50-60°C.

The essence of the claimed invention is as follows.

The claimed sulfur fullerene derivatives described by the general formula 1 are a new and previously unknown class of compounds of fullerenes. It is assumed that these fullerene derivatives will be used in pharmaceutical and medical research as a biocompatible form of the fullerene. At the same time, they can be promising in the use as an organic photovoltaic material for organic solar batteries.

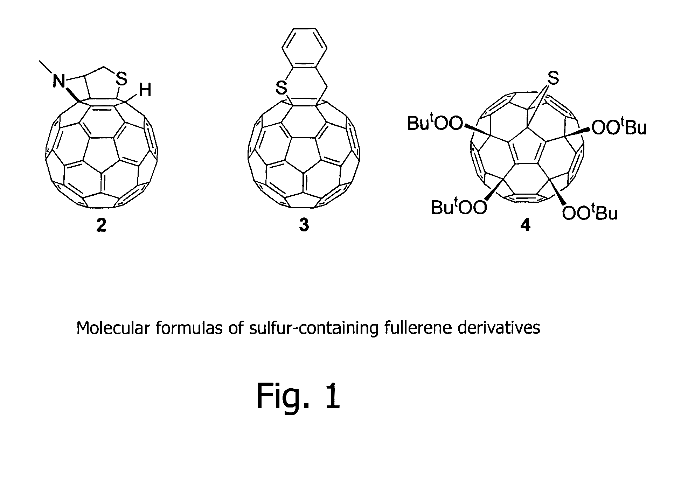

Currently, there is known not a lot of sulfur-containing fullerene derivatives in which there is a covalent bond between the sulfur atoms and the carbon atoms of the fullerene skeleton. As examples of such derivatives the product of intramolecular thiol attachment to the double bond of C₆₀ 2 can be given [M. Izquierdo, S. Osuna, S. Filippone, A. Martin-Domenech, M. Sola, N. Martin. Eur. J. Org. Chem., 6231-6238 (2009)], compound **3,** which is formed as a result of the Diels-Alder reaction [M. Ohno, S. Kojima, Y. Shirakawa, S. Eguchi. Tetrahedron Lett., 36, 6899 (1995)], and also compound **4,** which has a thiirane cycle [X. Yang, S. Huang, Z. Jia, Z. Xiao, Z. Jiang, Q. Zhang, L. Gan, B. Zheng, G. Yuan, S. Zhang. J. Org. Chem., 73, 2518-2526 (2008)] (Fig. 1). However, all of these fullerene derivatives are difficult considered to be close structural analogues of sulfur-containing fullerene derivatives of general formula 1 as they contain only one sulfur atom covalently linked to a fullerene skeleton, and they are the products of cyclization or cycloaddition reactions.

The closest structural analogues of the claimed sulfur fullerene derivatives are previously described mixtures of compounds of the general formula C₆₀(SR')ₙ with unknown composition and structure [Y. Takaguchi, Y. Katayose. Y. Yanagimoto, J. Motoyoshiya, H. Aoyama, T. Wakahara, Y. Maeda, T. Akasaka. Chem. Lett., 32, 1124 (2003) (n=2), 26 - L.Y. Chiang, United States Patent "Polyorganofullerenes", Nº 6046361 of 4.04.2000 (n=2-30)].

There are several fundamental differences between the claimed compounds of the general formula 1 and the earlier known analogues. First, the compounds of general formula 1 have clearly stated structure with five fragments of the SR, attached to the fullerene skeleton around a five-membered ring. Second, the structure **1** implements cyclopentadienyl ligands attachment motif to the skeleton of fullerene C₆₀ instead motifs [1, 2] for joining [6,6]fullerene communication skeleton as in C₆₀(SR')₂ or randemized polyaddition as in C₆₀(SR')ₙ. Third, the compounds of general formula **1** contain in its structure additional fragment X, which is responsible for the manifestation by the compounds of formula 1 of specific properties.

Synthesis methods compounds are most different. So, it is known that C₆₀(SR')₂ compounds are prepared by photochemical addition reaction of disulfides to the fullerene skeleton, flowing in the presence of diphenyl diselenide as a radical reaction initiator. This method is applicable to only a limited range of substrates and is characterized by low yields of the desired reaction product. Polymercaptofullerenes C₆₀(SR')ₙ are prepared by reacting polysulfone-(C₆₀(SO₄₎ₙ₎ or polynitrofullerenes (C₆₀(NO₂₎ₙ₎ with mercaptans, thiols or thioacids flowing in the presence of a base - triethylamine or 1.8-diazabicyclo[5.4.0]undecene-7 (DBU). The resulting compounds are complex mixtures of products with unknown composition and structure.

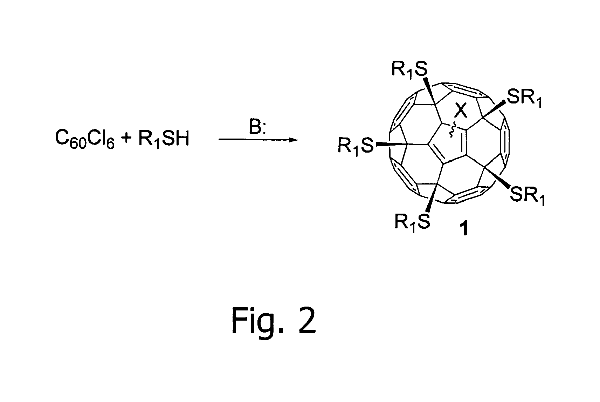

The inventive method for producing sulfur-containing fullerene derivatives of general formula 1 is based on reacting the C₆₀Cl₆ chlorfullerene with derivatives of thioacids, thioacids and thiols in the presence of a base (Fig. 2). As the base there may be used carbonates and alkali metal bicarbonates, tertiary amines such as triethylamine, as well as any other compounds which can be used as the term "base" in view of their ability to bind HCl released in the reaction. The best results were obtained using sodium acetate as a base.

The principal difference of this invention is the use of thiols and similar compounds as reagents for functionalization of chlorfullerene C₆₀Cl₆. The advantages of the claimed method for producing sulfur-containing fullerene derivatives may also include the use of a mild base (sodium acetate or potassium carbonate), the flow speed of synthesis (1 h) and high yields of products (60% to 75%).

Reactions proceed most smoothly at a temperature of 50-60° C and, as a rule, with 100% selectivity leads to the formation of sulfur-containing fullerene derivatives of general formula **1.** In the case of poor quality of the reagent or the characteristics of its structure the reaction proceeds with less selectivity, the desired product can be isolated in pure form with a yield of more than 60% by chromatographic purification. Reaction of chlorfullerene C₆₀Cl₆ with the alcohols in the presence of corresponding sodium alcoholate as a base leading to the formation of fullerene **5** alkoxy derivatives with low yields is shown in fig.3.

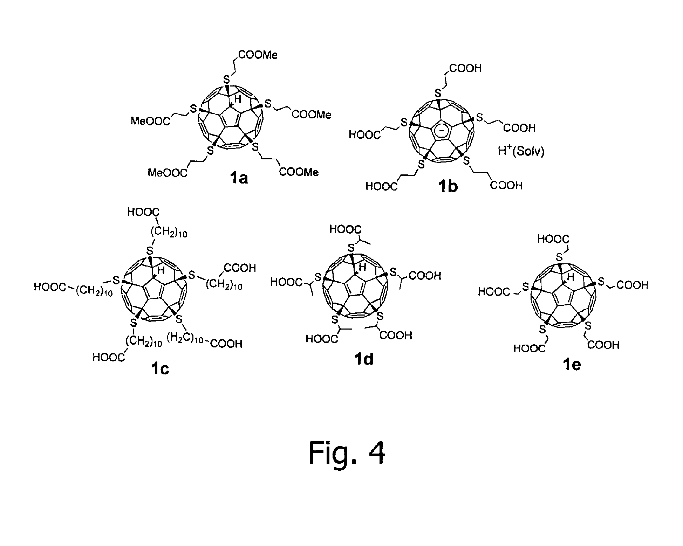

The developed approach allows receiving a variety of fullerene derivatives. Some examples of the synthesized compounds are presented in Fig. 4. Reaction of chlorfulleren C₆₀Cl₆ with thioacid esters proceeds in the presence of excess base - potassium carbonate or sodium acetate, with heating a reagent solution in toluene for 1-2 hours. Isolation of the reaction products is carried out by column chromatography on silica gel using as eluent a mixture of toluene and acetonitrile. Thus, the compound **1a** (Example 1), which is converted by acid hydrolysis into the polycarboxylic derivative **Ib** was obtained.

As reagents there were successfully used thioacids, allowing thereby one-step synthesis of polycarboxylic derivatives **Ib-1e** (Examples 2 - 4), which salts are characterized by high water solubility (> 50 mg / ml). The reaction with thioacids proceeds in the presence of excess base, sodium acetate under heating. As reaction proceeds, sodium salts of the product precipitate from the reaction mixture, which are then separated by filtration, dissolved in distilled water. From the aqueous solution compound **Ib-1e** are precipitated by addition of hydrochloric acid. The developed method of synthesis allows easily and efficiently producing polycarboxylic fullerene derivatives in multigram quantities.

It should be also noted that depending on the structure of thioacid or ester thereof, rigidity of the base used and the reaction conditions, the resulting compounds may contain a chlorine atom as a sixth ligand attached to the fullerene skeleton, or a hydrogen atom or a hydroxyl group or a negative charge delocalized the cyclopentadienyl moiety on the fullerene skeleton. In the latter case there are formed fullerene anions such as **Ib**, which are extremely stable and not protonated in an aqueous medium even under the action of organic acids (acetic acid, trifluoroacetic acid). Similar stable fullerene anions are unique and can find practical applications, particularly in non-linear optics, and for producing materials with unusual magnetic properties [28 - P.W. Stephens, D. Cox, J. W. Lauher, L. Mihaly, J. B. Wiley, P. -M. Allemand, A. Hirsch, K. Holczer, Q. Li, J. D. Thompson, F. Wudl. Nature, 355, 331 (1992)].

The composition and structure of the compounds obtained were proved using a complex of physical and chemical methods of investigation: NMR spectroscopy on ¹H and ¹³C nuclei, the two-dimensional correlation spectroscopy, infrared spectroscopy, optical absorption spectroscopy and elemental analysis. Purity of compounds was confirmed by using high effective liquid chromatography. Discussion of all received spectral and analytical data is impossible in the framework of this application. So the data for most typical compounds are given.

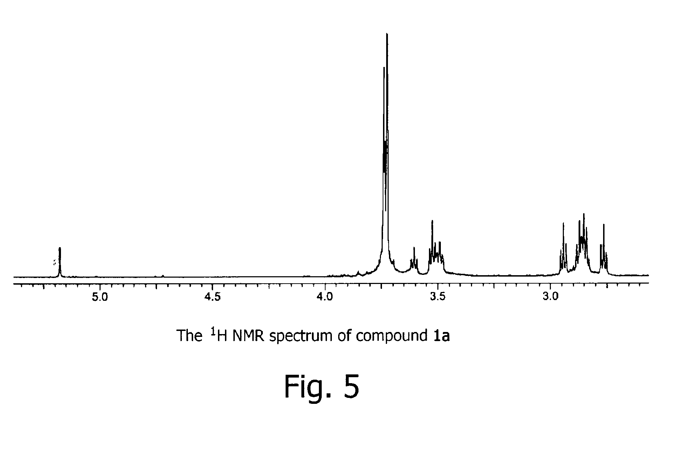

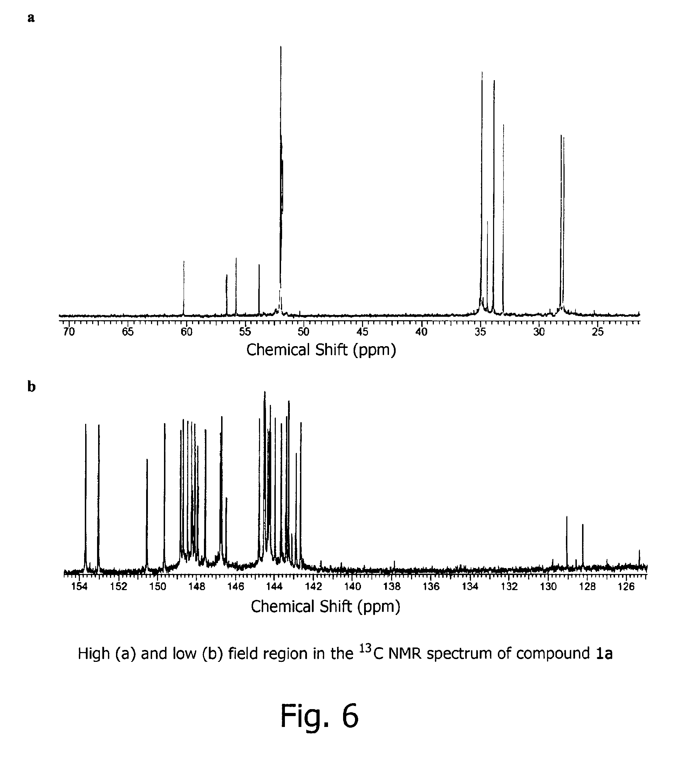

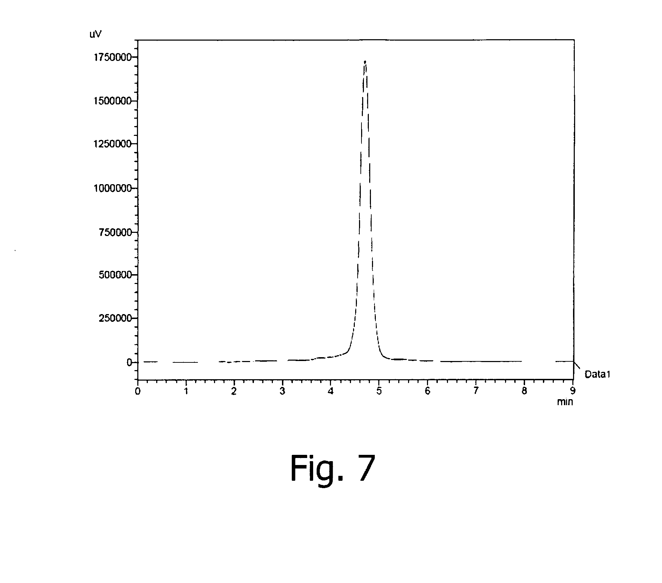

In FIG. 5 and FIG. 6 there are given the NMR spectra of compound **1a**, which confirm its Cₛ-symmetric structure. In the ¹³C NMR spectrum of compound **1a** detected are 28 signals corresponding to the sp²-hybrid carbon atoms of the fullerene skeleton, 3 signals corresponding to the carboxyl carbon atoms (total 31 signals in low fields), 4 signal from sp³-hybrid carbon atoms of the fullerene skeleton, and 9 - from sp³-hybrid atomic ligands (total 13 signals in the high fields). This spectrum is completely consistent with the molecular structure of compound **1a**. The ¹H NMR spectrum of compound **1a** has a complex structure of partially overlapping signals from the three types of nonequivalent symmetrically-S-CH₂CH₂COOMe groups. However, the integrated intensities of the signals observed and their multiplicity are generally consistent with the proposed structure. High purity of the obtained product **1a** is confirmed by chromatogram given in Fig. 7.

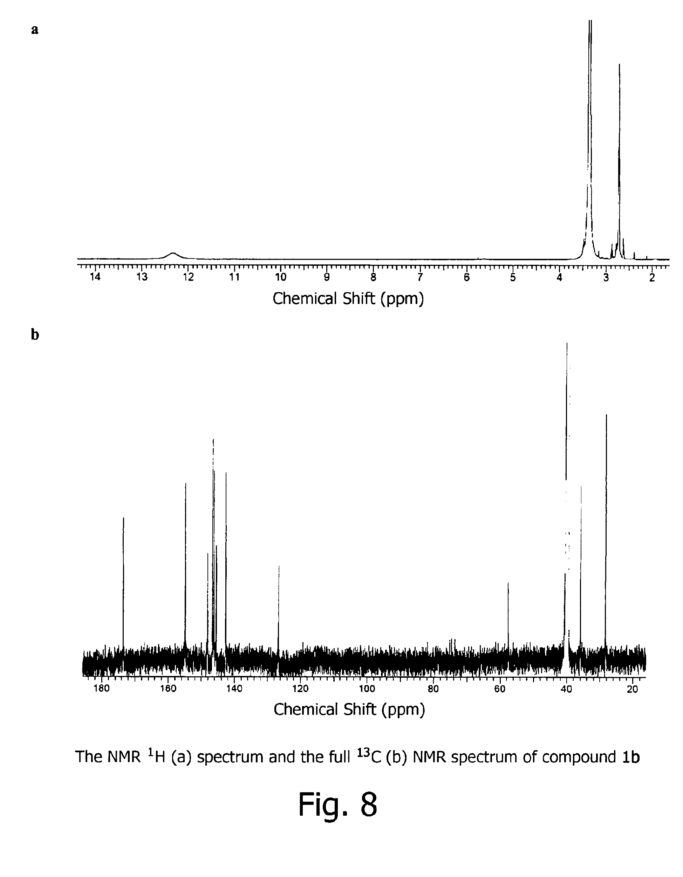

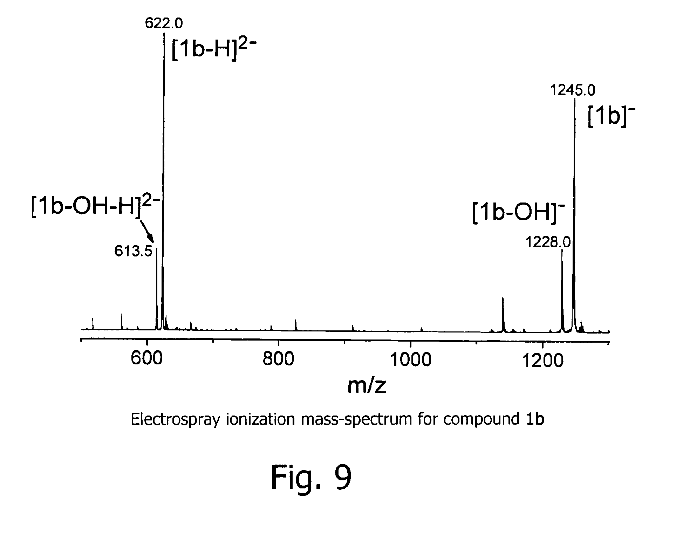

In FIG. 8 represented are the NMR spectra of compound **1b.** This compound represents stable in air fullerene anion, the only structural analogy of which are salt anions received by us [C₆₀(CN)₅]⁻ [P.A. Troshin, E. A. Khakina, A. S. Peregudov, D. V. Konarev, I. V. Soulimenkov, S. M. Peregudova, and R. N. Lyubovskaya, Eur. J. Org. Chem., 2010, 3265-3268]. The ¹H NMR spectrum of compound **1b** contains only the signals belonging to the five symmetry-equivalent residues of 3-mercaptopropanoic acid. Please, note that one of the signals at 3.37 ppm overlaps with the signal of the water, present in DMSO-D6. Weak field region in the ¹³C-NMR spectrum has a peak at 173 ppm, corresponding to the carboxyl group (-C(O)OH), and seven signals belonging to the fullerene skeleton. Strong field region is represented by two signals from sp³-hybrid carbon atoms of symmetry-equivalent residues of 3-mercaptopropanoic acid and a signal from the five symmetry-equivalent sp³-hybrid carbon atoms of the fullerene skeleton. Thus, the molecule of **1b** has symmetry C₅ᵥ unique for fullerene compounds, and the fivefold symmetry axis passes through the cyclopentadienyl moiety localized on the fullerene skeleton. Analysis of the compound **1b** by the HPLC method was impossible in view of its tight binding to the sorbent. Mass spectrum of the compound **1b** showed a signal corresponding to anion **[1b]**⁻ at m/z = 1245, fragmentary ion peak **[1b-OH]⁻** at m/z = 1228, and the peaks corresponding to dianions **[1b-H]**²⁻ (m/z = 622) and **[1b-H-OH]²⁻** (m/z = 613.5) (Fig. 9). Thus, the data of mass spectrometry completely confirm the composition of the compound **1b.**

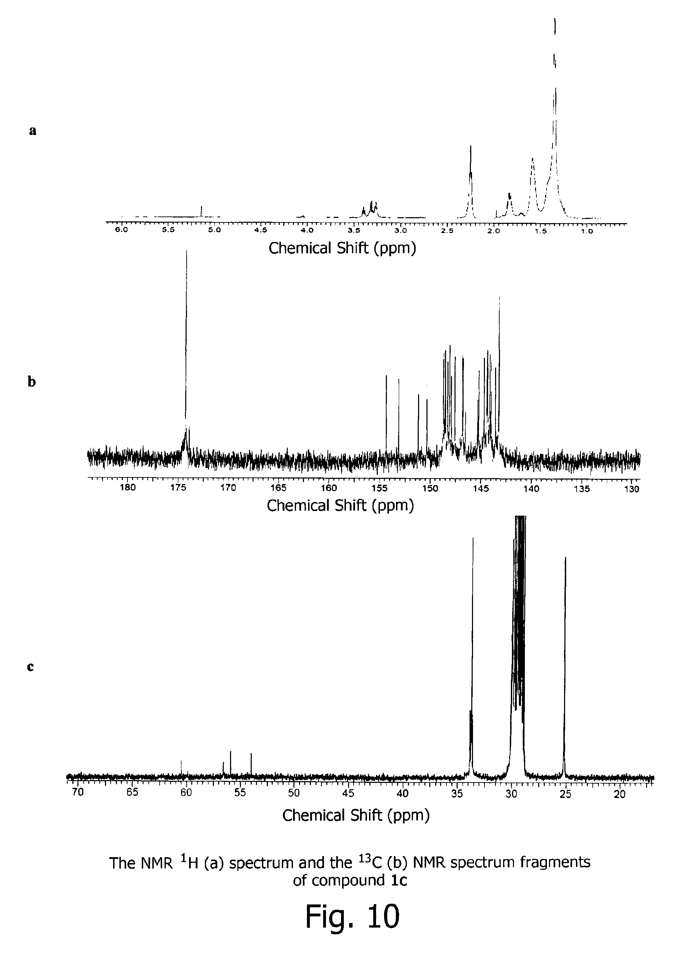

In FIG. 10 given are the ¹H NMR spectra and ¹³C compound **1c** containing five residues of 11-mercaptoundecanoic acid. The ¹H NMR spectrum exhibits a signal of hydrogen atoms attached to the fullerene skeleton, as a singlet at 5.15 ppm. There is also a group of signals at 3.31 ppm attributing to the methylene units, directly neighboring the carboxyl groups in the compound **1c**. At 2.26 ppm a multiplet is observed, which can be ascribed to the methylene groups neighboring the sulfur atoms attached to the fullerene skeleton. Also observed are signals of methylene protons at 1.36, 1.60 and 1.84 ppm. The ¹H NMR spectrum with the signals by number and the observed chemical shifts is fully consistent with the molecular structure of compound **1c**.

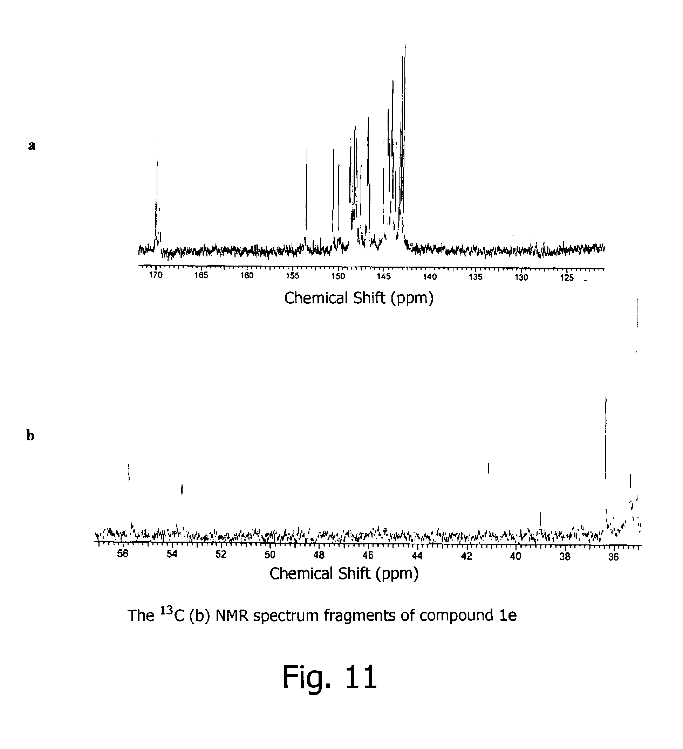

FIG. 11 depicts the ¹³C NMR spectrum of compound **1e**, containing five residues of mercaptoacetic acid. In the given spectrum, there are 28 signals corresponding to sp²-hybrid carbon atoms of the fullerene skeleton, 3 signals corresponding to the carboxyl carbon atoms, 4 signals from sp³-hybrid carbon atoms of the fullerene skeleton and 3 signals belonging to sp³-hybrid carbon atoms of ligands. Thus, the spectrum fully confirms the Cₛ-symmetrical structure of compound **1e.**

The claimed invention is illustrated, but not limited at all to, the following examples.

### Example 1. Synthesis of compound 1 a.

Chlorfullerene C₆₀Cl₆ (500 mg, 0.5 mmol) was dissolved in argon atmosphere in 200 ml of freshly distilled toluene. To a solution in the argon flow there was added 500 mg of dry sodium acetate crushed in a mortar and a large excess of methyl ether of 3-mercaptopropionic acid (1, 6 ml, 14.4 mmol). The reaction mixture was heated to 50-60°C with vigorously stirring for six hours. The extent of reacting was controlled by thin-layer chromatography on a silica gel plate. Isolation of the reaction products was performed by column chromatography on silica gel using as eluents mixtures of toluene and acetonitrile. Yield of the analytically pure (99 +%) product was 420 mg (64% of theory).

¹H NMR (600 MHz, CDCl₃, δ, ppm); 2.76 (t, 2H), 2.86 (m, 6H), 2.94 (t, 2H), 3.50 (m, 6H), 3.61 (t, 2H), 3.73 (m, 17H), 5.18 (s, 1H).

¹³C NMR (150 MHz, CDCl₃, δ, ppm); 27.95, 28.19, 33.08, 33.90, 34.94, 34.95, 51.93, 51.97, 51.99, 53.81, 55.80, 56.58, 60.27, 128.24, 129.05, 142.66, 142.90 , 143.10, 143.66, 143.98, 144.23, 144.29, 144.36, 144.52, 144.55, 144.80, 146.50, 146.73, 146.79, 147.57, 147.97, 148.12, 148.22, 148.28, 148.48, 148.72, 148.84, 149.68, 150.57, 153.04, 153.70.

### Example 2. Synthesis of compound 1b.

Chlorfullerene C₆₀Cl₆ (1500 mg, 1.6 mmol) was dissolved in argon atmosphere in 800 ml of freshly distilled toluene. To a solution there was added 5-10 g of dry sodium acetate crushed in a mortar and a large excess of 3 mercaptopropanoic acid (3.75 ml, 35 mmol). The reaction mixture was heated to 50-60° C with vigorously stirring for one hour. The reaction was accompanied by the formation of a reddish orange product precipitate. After completing the reaction, the precipitate was filtered, washed with acetone and then washed off the filter with distilled water. To the resulting bright red solution there was dropwise added hydrochloric acid until complete precipitation of the polycarboxylic derivative **1b.** The resulting bright red precipitate was separated by centrifugation, washed three times with distilled water and then transferred to a round bottom flask with acetone of 250ml and evaporated to dryness. The product yield was 1450 mg (72% of theory).

¹H NMR (600 MHz, (CD₃₎₂SO, δ, ppm): 2.73 (t, 1OH), 3.37 (t, 10H), 12.32 (br. s, 5H) ¹³C NMR (150 MHz, (CD₃₎₂SO, δ, ppm): 28.55, 36.20, 57.35, 127.10, 142.45, 146.10, 146.55, 147.12, 148.32, 155.17, 173.44.

### Example 3. The synthesis of compound 1c.

Chlorfullerene C₆₀Cl₆ (500 mg, 0.5 mmol) was dissolved in argon atmosphere in 400 ml of freshly distilled toluene. To the solution there was added 3-5 g of dry sodium acetate crushed in a mortar and a large excess of 11-mercaptoundecanoic acid (1462 mg, 7 mmol). The reaction mixture was heated to 50-60°C with vigorously stirring for one hour. The reaction was accompanied by the formation of reddish orange product precipitate. After completing the reaction, the precipitate was filtered, washed with acetone and then washed off the filter with distilled water. To the resulting bright red solution there was dropwise added hydrochloric acid until complete precipitation of polycarboxylic derivative **1c**. The resulting bright red precipitate was separated by centrifugation, washed three times with distilled water and then transferred to a round bottom flask with acetone of 250ml and evaporated to dryness. The yield of product was 650 mg (72% of theory).

¹H NMR (600 MHz, (CD₃₎₂CO:CS₂ (1:1), δ, ppm): 1.36 (m, 50 H), 1.60 (m, 20 H), 1.84 (m, 10H), 2.26 (m, 10 H), 3.31 (m, 10H), 5.15 (s, 1H.).

¹³C NMR (150 MHz, (CD₃₎₂CO:CS₂ (1:1), δ, ppm): 25.19, 29.52, 29.63, 29.67, 29.77, 29.08, 30, 30.05, 30.09, 33.6, 33.71, 33.87, 53.99, 55.91, 56.59, 60.50, 143.19, 143.22, 143.28, 143.60, 144.01, 144.12, 144.17, 144.34 , 144.39, 144.43, 144.69, 145.24, 145.34, 146.77, 146.85, 147.60, 147.95, 148.12, 148.30, 148.51, 148.59, 148.71, 150.35, 151.22, 153.15, 154.34, 174.26.

### Example 4. Synthesis of compound 1e.

Chlorfullerene C₆₀Cl₆ (1006 mg, 1.1 mmol) was dissolved in argon atmosphere in 700 ml of freshly distilled toluene. To a solution there was added 5-10 g of dry sodium acetate crushed in a mortar and a large excess of mercaptoacetic acid (3 ml, 30 mmol). The reaction mixture was heated to 50-60°C with vigorously stirring for one hour. The reaction was accompanied by the formation of a reddish orange product precipitate. After completing the reaction, the precipitate was filtered, washed with acetone and then washed off the filter with distilled water. To the resulting bright red solution there was dropwise added hydrochloric acid until complete precipitation of the polycarboxylic derivative **1e.** The resulting bright red precipitate was separated by centrifugation, washed three times with distilled water and then transferred to a round bottom flask with acetone of 250 ml and evaporated to dryness. The yield of product was 818 mg (65% of theory).

¹H NMR (600 MHz, (CD₃)₂CO:CS₂ (1:1), δ, ppm): 4.24 (s, 4H), 4.27 (s, 2H), 4.35 (s, 4H)

¹³C NMR (150 MHz, (CD₃)₂CO:CS₂ (1:1), δ, ppm) 35.16, 35.47, 36.41, 41.15, 55.78, 53.63, 56.71, 142.96, 143.13, 143.20, 143.34, 143.81 , 144.09, 144.17, 144.21, 144.25, 144.49, 144.69, 145.12, 146.59, 146.80, 146.87, 147.02, 147.57, 148.00, 148.12, 148.32, 148.47, 148.67, 148.77, 150.03, 150.64, 153.54, 153.70, 169.60, 169.88 , 170.01.

Thus, the claimed technical solution offers a new class of sulfur-containing fullerene derivatives of general formula **1** for the expansion of the arsenal of fullerene derivatives. A method of obtaining thereof provides the yield of the desired product of the reaction above 60 percent. Also high reaction rate (less than one hour) may be noted.

## Claims

1. Sulfur-containing fullerene derivatives of general formula 1, as follows: 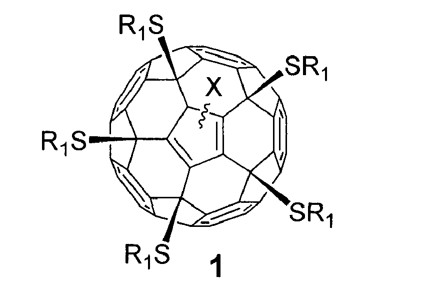 wherein in general formula 1, X is, but not limited to:
- negative charge («-»), which is localized on the fullerene skeleton, or
- a chlorine atom (-Cl) connected to the carbon skeleton, or
- a nitro group (-NO₂₎ or nitrate group (-ONO₂), attached to the carbon skeleton, or
- a hydrogen atom (-H), a linear or branched alkyl (CₙH₂ₙ₊₁; n=1-20), alkenyl (CₙH₂ₙ₋₁; n=1-20) or alkynyl radical (CₙH₂ₙ₋₃; n=1-20), attached to the fullerene skeleton or
- an alkoxy-O-R or thiolate moiety S-R, connected to the fullerene skeleton, wherein R is a hydrogen atom or a linear or branched alkyl (CₙH₂ₙ₊₁; n=1-20), alkenyl (CₙH₂ₙ₋₁; n=1-20) or alkynyl radical (CₙH₂ₙ₋₃; n=1-20), or
- a thioacid residue -S(CH₂)ₙCOOH or an ester thereof -S(CH₂)ₙCOOR, or an amide - S(CH₂)ₙCONR¹R², attached to the fullerene skeleton, where n=1-20, and R, R¹ and R² are hydrogen atoms or linear or branched alkyl (CₘH₂ₘ₊₁; n=1-20), alkenyl (CₘH₂ₘ₋₁; n=1-20) or alkynyl (CₘH₂ₘ₋₃, n=1-20) radicals;
wherein in the general formula 1 the SR¹ fragment is described, but not limited to the following definitions:
- the residue of thioacid or ester thereof, wherein R¹ is given as XCOOR; X is a linear or branched alkylene (CₙH₂ₙ; n=1-20), alkenylene (CₙH₂ₙ₋₂, n=1-20) or alkynylene (CₙH₂ₙ₋₄, n=1-20) radical, as well as saturated (CₙH₂ₙ₋₂, n=3-8), unsaturated (CₙH₂ₙ₋₄; n=5-8, CₙH₂ₙ₋₆; n=5-8, CₙH₂ₙ₋₈; n=8) or an aromatic ring; R is a hydrogen atom (-H), a linear or branched alkyl (CₙH₂ₙ₊₁; n=1-20), alkenyl (CₙH₂ₙ₋₁; n=1-20) or alkynyl radical (CₙH₂ₙ₋₃; n=1-20);
- the residue of the thioacid amide, where R¹ is given as XCONRR'; X is a linear or branched alkylene (CₙH₂ₙ; n=1-20), alkenylene (CₙH₂ₙ₋₂; n=1-20) or alkynylene (CₙH₂ₙ₋₄; n=1-20) radical, as well as saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or an aromatic ring; R and R'are hydrogen atoms or linear or branched alkyl (CₙH₂ₙ₋₁; n=1-20), alkenyl (CₙH₂ₙ₋₁; n=1-20) or alkynyl (CₙH₂ₙ₋₃, n=1-20) radicals;
- the residue of the thiol, where R¹ is a hydrogen atom or a linear or branched alkyl (CₙH₂ₙ₊₁; n=1-20), alkenyl (CₙH₂ₙ₋₁; n=1-20) or alkynyl radical (CₙH₂ₙ₋₃; n=1-20), and a saturated (CₙH₂ₙ₋₂; n=3-8), unsaturated (CₙH₂ₙ₋₄; n=5-8, CₙH₂ₙ₋₆; n=5-8, CₙH₂ₙ₋₈, n=8) or an aromatic ring;
- moiety of the general formula -S(CH₂)ₙSR, wherein R is a hydrogen atom or a linear or branched alkyl (CₙH₂ₙ₊₁; n=1-20), alkenyl (CₙH₂ₙ₋₁; n=1-20), or alkynyl radical (CₙH₂ₙ₋₃; n=1-20), and saturated (CₙH₂ₙ₋₂; n=3-8), unsaturated (CₙH₂ₙ₋₄; n=5-8, CₙH₂ₙ₋₆; n=5-8, CₙH₂ₙ₋₈; n = 8) or an aromatic ring;
- moiety of the general formula -S(CH₂₎ₙOR, wherein R is a hydrogen atom or a linear or branched alkyl (CₙH₂ₙ₊₁; n=1-20), alkenyl (CₙH₂ₙ₋₁; n=1-20) or alkynyl radical (CₙH₂ₙ₋₃; n=1-20), and a saturated (CₙH₂ₙ₋₂; n=3-8), unsaturated (CₙH₂ₙ₋₄; n=5-8, CₙH₂ₙ₋₆; n=5-8, CₙH₂ₙ₋₈; n = 8) or an aromatic ring;
- moiety of the general formula -S(CH₂)ₙHal, where Hal is F, CI, Br or I;
- moiety of the general formula -S(CH₂₎ₙNRR' or -S(CH₂₎ₙN⁺RR'R"X⁻, wherein R, R' and R" are hydrogen atoms or linear or branched alkyl (CₙH₂ₙ₊₁; n=1-20), alkenyl (CₙH₂ₙ₋₁; n=1-20) or alkynyl (CₙH₂ₙ₋₃; n=1-20) radicals, as well as saturated (CₙH₂ₙ₋₂; n=3-8), unsaturated (CₙH₂ₙ₋₄; n=5-8, CₙH₂ₙ₋₆; n=5-8, CₙH₂ₙ₋₈; n=8) or aromatic rings; X⁻ is any anion.

2. A process for preparing sulfur-containing fullerene derivatives of general formula 1 according to claim 1, comprising reacting a C₆₀Cl₆ chlorfullerene with a nucleophile agent in the presence of a base, **characterized in that** as the nucleophile agent derivatives of thioacids, thioacids or thiols are taken.

3. The process for preparing according to claim 2, **characterized in that** the process is carried out at a temperature of 0-150°C, preferably at 50-60°C.
